# EUROPEAN PATENT SPECIFICATION

(11) **EP 2 023 838 B1**
(45) Date of publication and mention of the grant of the patent: **02.07.2014**
(21) Application number: 07732933.2
(22) Date of filing: 21.05.2007
(51) Int. Cl.: A61B 17/80, A61B 17/70

(54) **Bone fixation device**
Knochenfixationsvorrichtung
Dispositif de fixation osseuse

(30) Priority: 26.05.2006 GB 0610630
(43) Date of publication of application: 18.02.2009
(73) Proprietor: Cunliffe, Mark, Richard, Edgerton Huddersfield West Yorkshire HD1 5QB (GB); Ness, Malcolm, Graham, Longframlington Morpeth Northumberland NE65 9EB (GB)
(72) Inventor: Cunliffe, Mark, Richard, Edgerton Huddersfield West Yorkshire HD1 5QB (GB); Ness, Malcolm, Graham, Longframlington Morpeth Northumberland NE65 9EB (GB)
(74) Representative: McDonough, Jonathan
(86) International application number: PCT/GB2007/001912
(87) International publication number: WO 2007/138270

(56) References cited:
- EP-A- 0 491 983
- DE-U1- 9 004 960
- GB-A- 2 405 342
- US-A- 6 022 350
- US-A1- 2003 074 004
- US-A1- 2003 153 912
- US-A1- 2004 111 089
- US-A1- 2005 015 090
- US-A1- 2005 065 515
- US-A1- 2006 009 771
- US-B1- 6 730 091

## Description

The present invention relates to a bone fixation device. More particularly, the present invention relates to a bone fixation device comprising first and second screw receiving members connected together by a relatively pliable connecting arm adapted to bend before the screw receiving members.

Bone fixation plates are known. Such plates typically comprise a plastically deformable plate such as a metal plate. A plurality of apertures extend through the plate. In use the plate is positioned against a bone and screws passed through the apertures to fix the plate to the bone.

In order to ensure a close fit between the plate and the bone it is often necessary to deform the plate slightly. Deforming the plate distorts the apertures, this reduces the accuracy of the fit between plate and screws and may allow the plate to move relative to the bone during use.

A further bone fixation device is disclosed in PCT/US2005/020157. Such a device comprises a number substantially spherical screw receiving members having screws extending therethrough. Extending from each screw receiving member is a connecting member. A connecting arm, rather than extending along a long axis between the screw receiving members, extends to the side of the long axis. The screw receiving members are connected to the connecting arm by the connection members. Such a construction has a number of drawbacks. It is complex having a number of parts which must be assembled in a multistep procedure. In addition, one cannot simply twist the device about the long axis as is often required.

US 6022350 discloses a bone fixing device comprising an elongate link means receiving at least one bone fastening screw which passes through an orifice formed in the link means. In the bottom of the link means there is included a bearing surface of essentially circular cross section. The head of the screw includes an essentially spherical surface for bearing against said bearing surface. The link means include a first thread in the vicinity of said orifice. The device further includes a plug having a second thread suitable for cooperating with the first thread, the plug being suitable for coming into clamping contact against said screw head to hold it in a desired angular position.

US 6, 730, 091 B1 discloses a bone fixation device according to the preamble of claim 1.

The device according to the invention seeks to overcome these problems.

Accordingly, the present invention provides a bone fixation device comprising
first and second screw receiving members, each member having an aperture extending therethrough for receiving a screw; and
a plastically deformable connecting arm extending between the screw receiving members along a long axis defined by the first and second screw receiving members;
the arm being more pliable than the screw receiving members such that (a) on twisting the device about its long axis the arm twists before the screw receiving members deform; and (b) on bending the device along its long axis the arm bends before the screw receiving members deform;
a part of at least one aperture being threaded for receiving a screw;
the bone fixation device further comprising at least one screw adapted for threaded engagement with the threaded portion of the aperture, the screw having a tapered head adapted to be drawn into the aperture to press fit with the walls of the aperture on threading the screw into engagement with the threaded portion of the aperture;
wherein the aperture is cylindrical.

The device according to the invention can be attached by a simplified procedure whereby it is deformed to the correct shape without deforming the apertures, placed against the bone and then screwed in place. In addition, it can be both bent along its long axis and twisted about its long axis to correctly align the device with the bone to the patient.

The device according to the invention is deformed to fit a bone the connecting arm(s) will deform before the screw receiving members. This allows the device to be deformed to the correct shape without the apertures becoming distorted.

Preferably, the device comprises a plurality of screw receiving members connected together in a line by connecting arms.

Preferably, at least one connecting arm is cylindrical in cross section.

At least one screw receiving member can be substantially spherical.

Preferably, a portion of the spherical member is flattened.

The flattened part can be centered about a mouth of the aperture and is in a plane normal to the axis of the aperture.

The cross section of the cylindrical aperture can be constant along its length.

The present invention will now be described by way of example only and not in any limitative sense with reference to the accompanying drawings in which
Figure 1 shows a known fixation plate before and after deformation;
Figure 2 shows a further known bone fixation device in perspective view;
Figure 3 shows a bone fixation device according to the invention in perspective view; and
Figure 4 shows the embodiment of Figure 3 in combination with a screw;

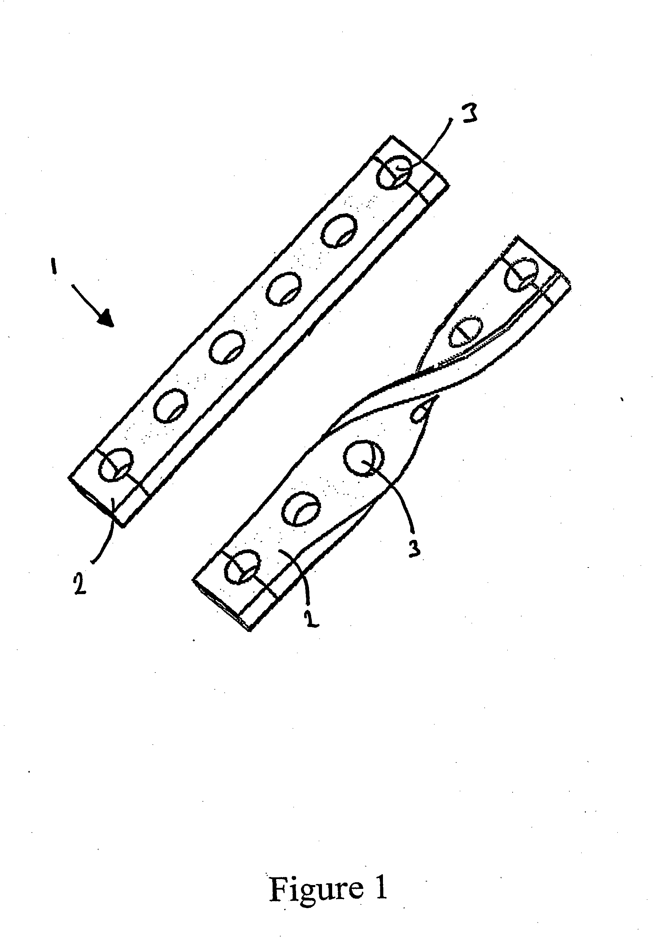

Shown in Figure 1 is a known bone plate 1. The bone plate 1 comprises a metal plate 2 having a plurality of apertures 3. In use the bone plate 1 is placed against a bone (not shown). Bone fixation screws (not shown) are passed through the apertures 3 and then screwed into the bone. The screw heads typically engage with the plate 1 firmly fixing the plate 1 in position and preventing it from being displaced with respect to the bone.

A problem can arise however if the bone plate 1 needs to be deformed before it can be fixed to the bone. Deformation of the plate 1 deforms the apertures 3 in the plate 1 as shown. This prevents the screw heads from accurately engaging with the apertures 3 which may result in the plate 1 being free to wobble slightly with respect to the bone. This can reduce the effectiveness of the bone plate 1 as a support for the bone.

In addition, it can be difficult to remove such a known bone plate 1 from the bone when it is no longer required. The screw heads tend to cold weld to the bone plate 1 over time making the screws difficult to remove. It is often necessary to cut the bone plate 1 free which can result in damage to the bone. Such a known bone plate 1 is also limited as to how it can be deformed. Whilst the plate 1 can be bent as shown in Figure 1 it is not a simple matter to twist it such that the apertures 3 lie in different planes.

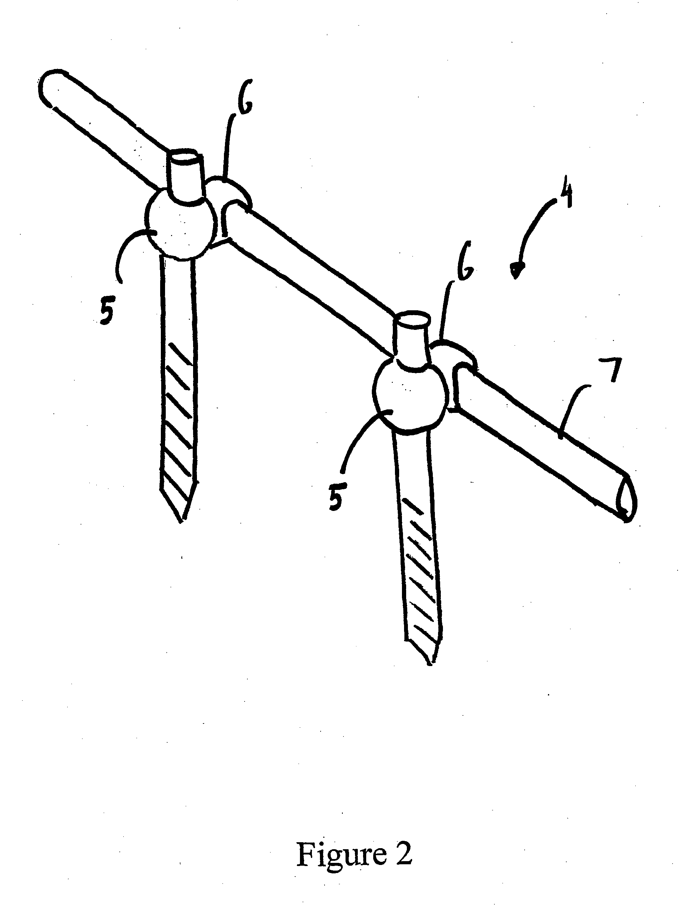

Shown in Figure 2 is a further known bone fixation device 4. This device 4 comprises a plurality of substantially spherical screw receiving members 5. Extending from each screw receiving member 5 is a connecting member 6. A connecting arm 7 extends through the connecting members 6 connecting the screw receiving members 5 together.

In use the screw receiving members 5 are arranged in the correct position and screwed to the bone. The connecting arm 7 is then passed through the connecting members 6 to complete the device 4.

Such a device 4 overcomes the problem of deformation of the screw receiving members by separating the screw receiving members 5 from the connecting arm 7 to be deformed. This separation however increases the complexity of the device 4 and the installation procedure. The connecting arm 7 needs to be deformed to a complex shape before it can be threaded through the connecting members 6. The threading can be difficult or even impossible in a confined space. Because of the gap between the screw receiving members 5 and the connecting arm 7 the device 4 is unsuitable for use where a longitudinal twist of the device 4 is required.

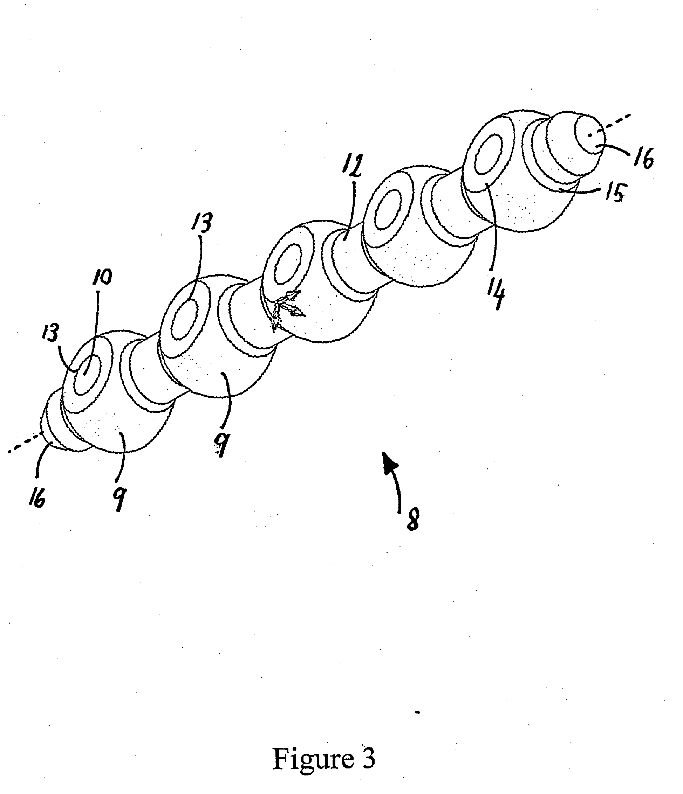

Shown in Figure 3 is a bone fixation device 8 according to the invention. The device comprises a plurality of screw receiving members 9. Apertures 10 extend through each of the screw receiving members 9 for receiving screws. A long axis 11 extends between each of the screw receiving members 9. Plastically deformable connecting arms 12 extend along the long axis 11 between the screw receiving members 9 as shown. In this embodiment the long axis 11 passes through the apertures 10 of the screw receiving members 9.

In use the bone fixation device 8 is gripped and bent to the required shape. The arms 12 are more pliable than the screw receiving members 9 and accordingly it is the arms 12 that bend when the force is applied, rather than the screw receiving members 9. The apertures 10 therefore remain undistorted. In addition, in contrast to known bone plates 1 a torsional (twisting) force can be applied to the device 8 rotating one or more of the screw receiving members 9 about the long axis 11 of the device 8 if required. As the long axis 11 passes along the length of the connecting arm 12 the connecting arm 12 twists about its length. The device 8 can therefore be twisted without significantly altering its dimensions. The device 8 can therefore be inserted into small apertures even after twisting.

In this embodiment of the invention each of the screw receiving members 9 is substantially spherical with the apertures 10 extending through the centres of the spheres 9. Each aperture 10 intersect the sphere at mouths 13 on opposite sides of the sphere 9. The sphere 9 comprises a slightly flattened portion 14 around one of the mouths. This reduces the profile of the device 8. It also provides an extended contact area between the screw receiving members 9 and the bone (not shown).

The connecting arms 12 between the screw receiving members 9 are cylindrical. The interface 15 between the arms 12 and spherical screw receiving members 9 is chamfered so that any bending or torsional forces do not concentrate at this interface 15.

The ends 16 of the device 8 are tapered as shown so that the device 8 can be placed between bone and soft tissue without surgically exposing the entire length of bone.

Each of the apertures 10 of this embodiment is cylindrical having a constant area along its length. A portion of the aperture 10 is threaded. The remainder of the aperture 10 is smooth walled.

After bending and/or twisting to the correct shape the device 8 is positioned against the bone. Screws (not shown) are inserted into the apertures 10 through the smooth portions and into threaded engagement with the threaded portions. On further rotation of the screws they penetrate and grip the bone, fixing the device 8 to the bone. A significant advantage of the device 8 is that it can be bent/twisted to the correct shape, positioned correctly and then screws inserted. This considerably simplifies the attachment procedure. As the screw receiving members 9 are aligned with the connection arms 12 along the long axis 11 the device 8 can be twisted about its length without any significant change in dimensions of the device 8. This is particularly useful when inserting the device 8 into small apertures.

The device 8 is adapted to be used with a screw (not shown) having two portions - a threaded portion for gripping the threaded portion of the aperture 10 and then the bone and a smooth portion extending from the threaded portion. The smooth portion has an outer face which is substantially cylindrical and of the same diameter as the threaded portion. The smooth portion is however slightly tapered with its diameter increasing in a direction away from the threaded portion. At its end the diameter of the smooth portion is slightly larger than the diameter of the aperture 10. As the screw is turned and is drawn into the aperture 10 the smooth portion of the screw abuts the smooth portion of the aperture 10 so producing a press fit.

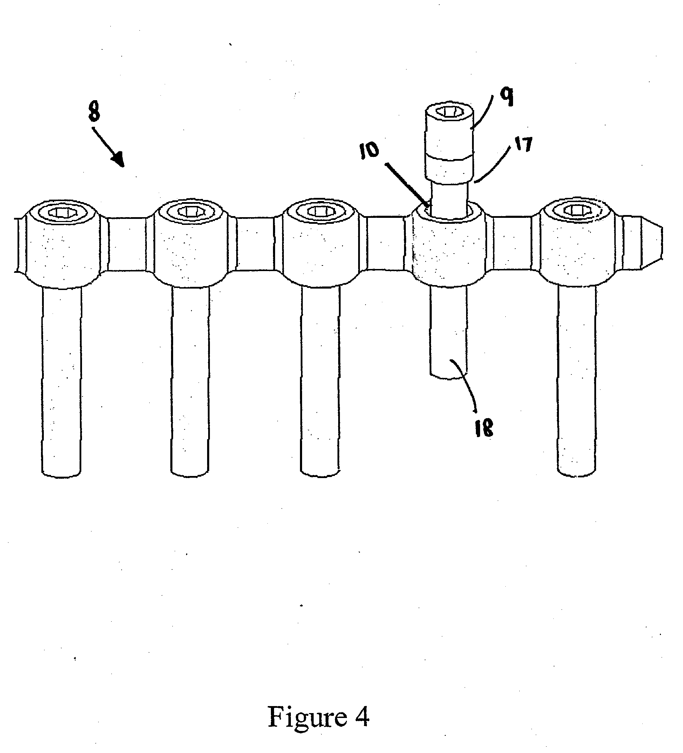

An alternative embodiment of the invention is shown in Figure 4. In this embodiment the threaded portion of the aperture 10 is narrower in diameter than the smooth portion. The screw 17 has a narrow threaded portion 18 and a wider smooth tapered head portion 19 as shown. The narrow threaded portion 18 of the screw 17 engages with the narrow portion of the aperture 10 drawing the larger smooth head portion 19 into abutment with the smooth portion of the aperture 10.

In a further embodiment of the invention the screw receiving members 9 are substantially elliptical.

The device 8 according to the invention can be used with any tool which grips the screw receiving members 9. One preferred embodiment of such a tool comprises jaws having cut out sections which match the spherical component of the screw receiving members 9. The tool also has cut outs which match the interface 15 between the spherical component 9 and connecting arm 12 so that the tool can apply a bending force throughout the length of the bone fixation device 8. In an alternative embodiment the jaws have cut outs which match the flattened portions 14 of the screw receiving members 9.

In use two of the tools are used to grip the screw receiving members 9. The device 8 is then bent and/or twisted to the desired shape and then released.

## Claims

1. A bone fixation device (8) comprising
first and second screw receiving members (9), each member (9) having an aperture (10) extending therethrough for receiving a screw; and
a plastically deformable connecting arm (12) extending between the screw receiving members (9) along a long axis (11) defined by the first and second screw receiving members (9);
the arm (12) being more pliable than the screw receiving members (9) such that (a) on twisting the device (8) about its long axis (11) the arm (12) twists before the screw receiving members (9) deform; and (b) on bending the device (8) along its long axis (11) the arm (12) bends before the screw receiving members (9) deform;
a part of at least one aperture (10) being threaded for receiving a screw;
the bone fixation device further comprising at least one screw adapted for threaded engagement with the threaded portion of the aperture (10), the screw having a tapered head adapted to be drawn into the aperture (10) to press fit with the walls of the aperture (10) on threading the screw into engagement with the threaded portion of the aperture (10);
**characterised in that** the aperture (10) is cylindrical.

2. A bone fixation device (8) as claimed in claim 1, comprising a plurality of screw receiving members (9) connected together in a line by connecting arms (12).

3. A bone fixation device (8) as claimed in either of claims 1 or 2, wherein at least one connecting arm (12) is cylindrical in cross section.

4. A bone fixation device (8) as claimed in any one of claims 1 to 3, wherein at least one screw receiving member (9) is substantially spherical.

5. A bone fixation device (8) as claimed in claim 4, wherein a portion (14) of the spherical screw receiving member (9) is flattened.

6. A bone fixation device (8) as claimed in claim 5, wherein the flattened part (14) is centred about a mouth (13) of the aperture (10) and is in a plane normal to the axis of the aperture (10).

7. A bone fixation device (8) as claimed in any one of claims 1 to 6, wherein the cross section of the cylindrical aperture (10) is constant along its length.

## Patentansprüche

1. Knochenfixiervorrichtung (8) umfassend
ein erstes und ein zweites Schraubenaufnahmeelement (9), wobei jedes Element (9) eine Öffnung aufweist, die sich zur Aufnahme einer Schraube durch das Element erstreckt;
einen plastisch deformierbaren Verbindungsarm (12), der sich entlang einer Längsachse (11), die von dem ersten und zweiten Schraubenaufnahmeelement (9) definiert wird, zwischen den Schraubenaufnahmeelementen (9) erstreckt;
wobei der Arm (12) verformbarer als die Schraubenaufnahmeelemente (9) ist, so dass (a) beim Verdrehen der Vorrichtung (8) um dessen Längsachse (11) der Arm (12) sich verdreht, bevor die Schraubenaufnahmeelemente (9) sich verformen; und (b) beim Biegen der Vorrichtung (8) entlang deren Längsachse (11) sich der Arm (12) biegt, bevor die Schraubenaufnahmeelemente (9) sich verformen;
wobei ein Teil wenigstens einer Öffnung (10) mit einem Gewinde zur Aufnahme einer Schraube versehen ist;
wobei die Knochenfixiervorrichtung ferner wenigstens eine Schraube umfasst, die für eine Ineingriffnahme mittels eines Gewindes mit dem mit einem Gewinde versehenen Abschnitt der Öffnung (10) ausgestaltet ist, wobei die Schraube einen verjüngt zulaufenden Kopf aufweist, der dazu ausgestaltet ist, in die Öffnung (10) gezogen zu werden, um eine Druckpassung mit den Wänden der Öffnung (10) einzugehen, wenn die Schraube in eine Ineingriffnahme mit dem mit einem Gewinde versehenen Abschnitt der Öffnung (10) gebracht wird;
**dadurch gekennzeichnet, dass** die Öffnung (10) zylindrisch ist.

2. Knochenfixiervorrichtung (8) nach Anspruch 1, umfassend eine Vielzahl von Schraubenaufnahmeelementen (9), die durch Verbindungsarme (12) in einer Linie mit einander verbunden sind.

3. Knochenfixiervorrichtung (8) nach Anspruch 1 oder 2, wobei wenigstens ein Verbindungsarm (12) einen zylindrischen Querschnitt aufweist.

4. Knochenfixiervorrichtung (8) nach einem der Ansprüche 1 bis 3, wobei wenigstens ein Schraubenaufnahmeelement (9) im Wesentlichen kugelförmig ist.

5. Knochenfixiervorrichtung (8) nach Anspruch 4, wobei ein Abschnitt (14) des kugelförmigen Schraubenaufnahmeelements (9) abgeflacht ist.

6. Knochenfixiervorrichtung (8) nach Anspruch 5, wobei der abgeflachte Abschnitt (14) um eine Mündung (13) der Öffnung (10) zentriert ist und in einer Ebene verläuft, die senkrecht zu der Achse der Öffnung (10) steht.

7. Knochenfixiervorrichtung (8) nach einem der Ansprüche 1 bis 6, wobei der Querschnitt der zylindrischen Öffnung (10) konstant entlang dessen Länge ist.

## Revendications

1. Dispositif (8) de fixation osseuse comprenant :
- des premier et second organes (9) de réception de vis, chaque organe présentant une ouverture (10) s'étendant à travers dudit organe pour recevoir une vis ; et
- un bras (12) de connexion déformable de manière plastique s'étendant entre les organes (9) de réception de vis le long d'un axe (11) défini par les premier et second organes (9) de réception de vis ;
- le bras (12) étant plus pliable que les organes (9) de réception de vis de sorte que (a) lors d'une torsion du dispositif (8) le long de son axe (11), le bras (12) se torde avant que les organes (9) de réception de vis ne se déforment ; et (b) lors d'une plicature du dispositif (8) le long de son axe (11), le bras (12) se plie avant que les organes (9) de réception de vis ne se déforment ;
- une partie d'au moins l'une des ouvertures (10) étant filetée pour recevoir une vis ;
- le dispositif (8) de fixation osseuse comprenant en outre au moins une vis adaptée pour s'engager de manière filetée avec la partie filetée de l'ouverture (10), la vis présentant une tête s'effilante adaptée à être prise dans l'ouverture (10) pour venir se plaquer contre les parois de l'ouverture (10) lors du vissage de la vis dans la partie filetée de l'ouverture (10) ;
**caractérisé en ce que** l'ouverture (10) est cylindrique.

2. Dispositif (8) de fixation osseuse selon la revendication 1, comprenant une pluralité d'organes (9) de réception de vis connectés ensemble en ligne par des bras de connexion (12).

3. Dispositif (8) de fixation osseuse selon l'une quelconque des revendications 1 ou 2, dans lequel au moins un bras (12) de connexion présente une section transversale cylindrique.

4. Dispositif (8) de fixation osseuse selon l'une quelconque des revendications 1 à 3, dans lequel au moins l'un des organes (9) de réception de vis présente une forme sensiblement sphérique.

5. Dispositif (8) de fixation osseuse selon la revendication 4, dans lequel une partie (14) de l'organe (9) de réception de vis sensiblement sphérique est aplatie.

6. Dispositif (8) de fixation osseuse selon la revendication 5, dans lequel la partie (14) aplatie est centrée autour d'une embouchure (13) de l'ouverture (10) et se trouve dans un plan orthogonal à l'axe de l'ouverture (10).

7. Dispositif (8) de fixation osseuse selon l'une quelconque des revendications 1 à 6, dans lequel la section transversale de l'ouverture (10) cylindrique est constante sur sa longueur.
